# FASCICULE DE BREVET EUROPEEN

(11) **EP 3 760 159 B1**
(45) Date de publication et mention de la délivrance du brevet: **25.10.2023**
(21) Numéro de dépôt: 20190987.6
(22) Date de dépôt: 30.08.2012
(51) Int. Cl.: A61C 7/00, A61C 19/045, A61B 5/00, A61B 6/14, A61C 9/00, A61B 5/11

(54) **PROCEDE DE CONCEPTION D'UN APPAREIL DENTAIRE**
VERFAHREN ZUR ENTWICKLUNG EINER ZAHNSPANGE
METHOD FOR DESIGNING A DENTAL APPARATUS

(30) Priorité: 31.08.2011 FR 1157669
(43) Date de publication de la demande: 06.01.2021
(62) Demande divisionnaire de: 12762340.3
(73) Titulaire: Modjaw, 69100 Villeurbanne (FR)
(72) Inventeur: JAISSON, Maxime, 73190 CHALLES LES EAUX (FR)
(74) Mandataire: Regimbeau

(56) Documents cités:
- EP-A1- 0 369 908
- WO-A2-2006/065955
- US-A- 4 836 778
- US-A1- 2008 057 466
- US-A1- 2008 176 182

## Description

L'invention concerne le domaine de la conception des orthèses pour la correction de dentitions, par des praticiens dentaires.

Elle concerne plus particulièrement un procédé de conception de telles orthèses, permettant d'améliorer leur adaptabilité et leur efficacité.

Le document US 2008/057466 A1 peut être considéré comme l'art antérieur le plus proche de l'invention telle que définie par les revendications ci-jointes.

Les orthèses ou dispositifs inter occlusaux sont des prothèses fixées (inlay, onlay, couronne, bridge), ou des gouttières occlusales, utilisées en thérapie musculaire (décontracter) ou articulaire (repositionner). Une gouttière est donc un outil orthopédique, qui doit assurer le retour à une position fonctionnelle. Cette branche de la dentisterie s'appelle l'occlusodontie. On utilisera dans la suite le terme d'appareil dentaire, pour couvrir tout objet devant être fabriqué et implanté dans une dentition, que ce soit pour remplacer une ou plusieurs dents ou parties de dents, ou pour s'ajouter à la dentition, par exemple se fixer dessus pour les déplacer.

La définition, et le positionnement d'une gouttière reposent donc autant sur une bonne définition des arcades dentaires, sur lesquelles elle s'appuie, que sur l'observation des mouvements mandibulaires.

Un système de FAO (fabrication assistée par ordinateur) de dispositifs inter occlusaux au sein d'un modèle numérique du massif facial, après captation du mouvement de la mandibule associe :
- une tomographie volumique à faisceau ouvert, conique - Cône Beam Computerized Tomography ou CBCT- des bases osseuses (mâchoires), qui donne une image de l'os de la mâchoire, et donc de l'articulation temporo-mandibulaire.
   ∘ la tomographie volumique à faisceau conique est utilisée en dentisterie afin d'obtenir une image en trois dimensions de l'articulation des mandibules, en raison d'une faible dosimétrie et de son caractère tridimensionnel : en une seule révolution, on balaye l'ensemble du volume à radiographier
- elle a fait l'objet d'une évaluation par la Haute Autorité de Santé en 2009
- un scanner optique pour obtenir un modèle virtuel des dents du patient
- un dispositif permettant de prendre en compte les mouvements de la mâchoire
- une caméra optique 3D apte à relever dans l'espace les points caractéristiques du massif facial, pour placer les arcades dentaires par rapport à ce massif facial.

Le dispositif inter occlusal est ensuite réalisé par frittage de poudres, ou par usinage, ou bien encore par stéréo-lithographie

On réalise un set-up orthodontique. Un set-up est une simulation du déplacement dentaire souhaité (état cible) en fin de traitement ; selon l'état de la technique il est obtenu par manipulation d'un modèle physique, ou peut être obtenu numériquement. Néanmoins les solutions proposées ne permettent pas actuellement de prévoir de manière fiable l'ensemble des conséquences sur l'environnement immédiat du déplacement d'une dent.

Dans l'état de la technique, l'anticipation du déplacement des racines se fait par modélisation physique, tandis que leur suivi nécessite des radios répétées, ou ne se fait pas (réticences à réaliser des examens d'imagerie chez l'enfant, du fait des effets secondaires des rayons).

La présente invention se propose de remédier à au moins une partie des inconvénients précités et propose une solution qui permette de prévoir de manière plus fiable les effets sur l'ensemble de la dentition d'une correction apportée à une dent, tout en limitant l'exposition du patient aux rayons.

A cet effet, l'invention concerne un procédé de conception d'un appareil dentaire selon la revendication 1 ci-jointe. Il s'agit d'un procédé de conception d'un appareil dentaire d'un massif facial comprenant des couronnes de dents et les racines leur correspondant, ledit procédé comprenant les étapes suivantes :
- constitution par imagerie médicale d'une image volumique du massif facial sous la forme d'un fichier numérique initial
- modification de ladite image volumique par traitement informatique dudit fichier numérique initial, et obtention d'un fichier numérique modifié, ladite modification comprenant au moins un déplacement de dent en vue d'une correction souhaitée de la dentition
- conception dudit appareil dentaire en utilisant ledit fichier numérique initial et ledit fichier numérique modifié, et production d'un fichier utilisable par des machines à commande numérique aptes à fabriquer un appareil dentaire.

Ledit procédé est particulier en ce que dans l'étape de modification de l'image volumique, pour chaque dent objet d'un déplacement, sa couronne est individualisée et il lui est associé la racine lui correspondant.

Ainsi le procédé selon l'invention est apte à prendre en compte les effets sur l'ensemble de la dentition, et permet donc d'obtenir des prévisions de comportement bien plus fiables.

Selon d'autres caractéristiques :
- ladite image volumique du massif facial peut comprendre l'image de l'articulation mandibulaire, de sorte à rendre compte des mouvements possibles de ladite articulation ; cela permet de prendre en compte les diverses positions possibles de la mâchoire pour la conception de l'appareil dentaire,
- ledit déplacement de dent peut être accompagné du tracé de courbes schématisant le positionnement des bords occlusaux de ladite dent et la position extrême des faces vestibulaires de ladite dent ; cela permet de facilement visualiser l'effet obtenu par la correction envisagée,
- ledit traitement informatique peut comprendre en outre l'évaluation des forces à appliquer à ladite dent pour permettre ledit déplacement, ce qui permet de prévoir de manière plus fiable les effets sur l'environnement immédiat,
- la mise en place de l'appareil dentaire peut être accompagnée de la mise en place de bracket en vue de fixer un fil à mémoire de forme, et ainsi guider la traction orthodontique ; cela permet d'améliorer la précision des corrections apportées,
- la mise en place de l'appareil dentaire peut être accompagnée de la mise en place d'arc en vue d'assister l'effort à appliquer à une arcade dentaire,
- la mise en place de l'appareil dentaire peut être accompagnée de la mise en place de minivis implantée au niveau de l'os ; la position de ces minivis peut alors être déterminée de manière optimale grâce à la connaissance des déplacements des racines de la dent ainsi que des dents voisines,

La présente invention concerne également l'application du procédé selon l'invention pour replacer correctement une ou plusieurs dents mal placées, pour remplacer une ou plusieurs dents manquantes, ou les restaurer par une ou des couronnes ou un ou des inlays, ou pour la conception d'une orthèse occlusale de libération occlusale ou d'antéposition mandibulaire.

La présente invention concerne enfin un système de conception d'un appareil dentaire d'un massif facial, spécialement conçu pour la mise en oeuvre du procédé selon l'invention, et comprenant un dispositif d'imagerie médicale, et un logiciel apte à traiter ladite image par des simulations de déplacement d'au moins une dent, et apte à créer un fichier exploitable par une machine à commande numérique pour fabriquer ledit appareil.

L'avantage découlant de la présente invention consiste en ce que la captation de mouvements améliore la définition de l'appareil dentaire, par exemple du dispositif inter occlusal (gouttière), mais aussi elle permet d'introduire la possibilité de détecter des pathologies articulaires, et de les traiter précocement grâce à des gouttières construites en FAO, à partir des données numériques recueillies directement sur le patient.

D'autres caractéristiques et avantages de l'invention ressortiront de la description détaillée qui va suivre se rapportant à un exemple de réalisation donné à titre indicatif et non limitatif, ainsi qu'aux dessins ci-après.
Figure 1: Placement du marqueur sur la surface cutanée objectivant le condyle gauche
Figure 2: Placement du marqueur sur la surface cutanée objectivant le condyle droit
Figure 3: Placement des marqueurs sur la surface cutanée objectivant l'épine nasale, le nasion et les deux points sous orbitaires.
Figure 4: Plan de francfort passant par les points de référence.

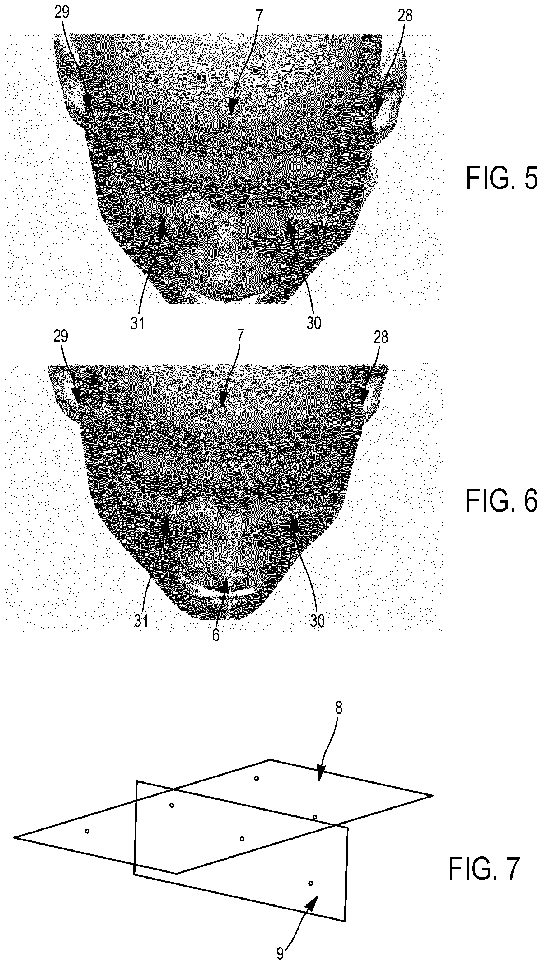

Figure 5: Point intercondylien. Le point est vu par transparence de la surface cutanée.
Figure 6: Axe sagittal médian passant par le point sous nasal, le point intercondylien et perpendiculaire au plan horizontal.
Figure 7 : Axe, points et plan de référence détectés par la caméra et objectivés par le logiciel. Les arcades dentaires seront situées par rapport à ces références.

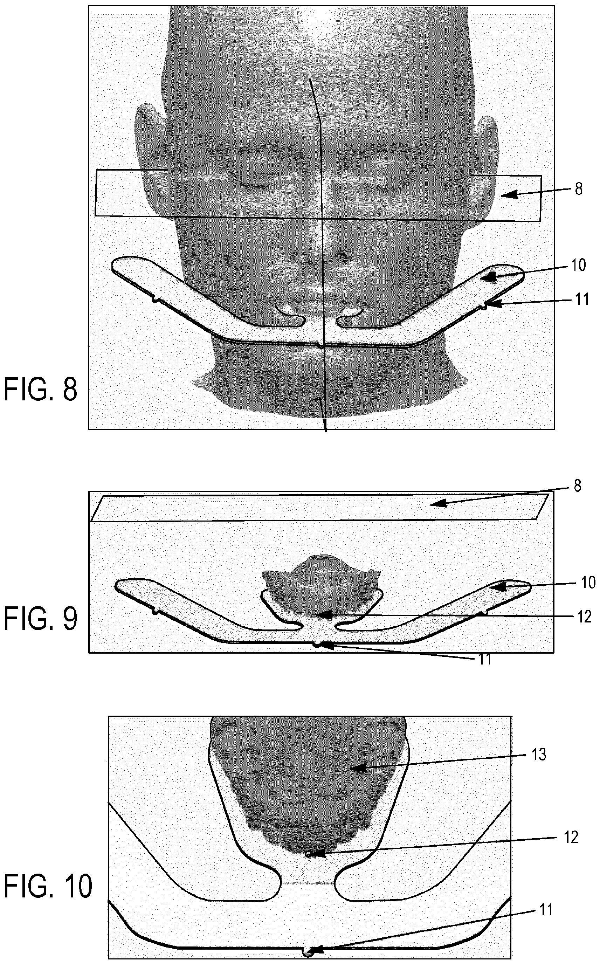

Figure 8: Type de caméra du commerce utilisable pour le procédé.
Figure 9: Plan de fox. Notre plaque de positionnement s'inspire de son design.
Figure 10: Plaque appliquée aux dents supérieures. Situation par rapport aux différents plans. Les repères ici sous forme de boules sont repérables par la caméra.
Figure 11: Vue en écorché du rapport entre la plaque et les dents maxillaires.
Figure 12 : Lors du placement en bouche de la plaque, le butoir se place au milieu incisif. Seule la boule repère en avant est visible par la caméra. La distance étant connue entre la boule et le butoir, la position du milieu incisif est ensuite déduite.
Figure 13: Empreinte laissée dans un matériau à déformation plastique sur la portion intrabuccale de la plaque. Cette portion est ensuite scannée. Le scanner enregistre également les bords de la plaque et des repères géométriques placés dessus Ici, un exemple de scanner de table.
Figure 14 : Corrélation nécessaire pour situer le modèle virtuel de l'arcade sur le modèle virtuel de l'empreinte laissée par les dents du patient sur la plaque.
Figure 15: Points déterminés sur l'arcade virtuelle ensuite recherché en bouche
Figure 16: Le stylet est suivi par la caméra et permet de cliquer sur les points d'intérêt préalablement choisis
Figure 17: Exemple de placement des diodes. Un écarteur est placé pour les rendre visibles par la caméra. La mandibule est mise en mouvement et enregistré.
Figure 18: Marquage des pointes cuspidiennes.
Figure 19: Création du plan d'occlusion sur l'arcade antagoniste
Figure 20 : Duplicata du maillage pour réaliser les surfaces de la gouttière.
Figure 21: Section par le plan cuspidien maxillaire de l'extrusion des bords.
Figure 22:Vue de la CfAO de la gouttière
Figure 23: Adaptation en bouche parfaite et conforme au projet informatique
Figure 24: Sphère de Monson. D'après Orthlieb.
Figure 25: Report des longueurs, situation du centre de la sphère de Monson sur le plan sagittal médian.
Figure 26: Vue de la sphère appliquée au modèle mandibulaire. Utilisation d'une calotte pour évaluer le positionnement dentaire.
Figure 27: La calotte anatomique ainsi obtenue nous donne une idée de la courbe de Spee en vue sagittale.
Figure 28: Situation d'un set up numérique par rapport à la "calotte occlusale dérivée de la sphère de Monson"
Figure 29: Importation des volumes radiculaires afin de valider le set up.
Figure 30: Correspondance des plans et axe préalablement déterminés par les caméras avec les articulateurs du commerce disponibles dans certains logiciels de CAO. Les arcades dentaires déjà situés par rapport à ces premiers sont donc facilement transférable sur les articulateurs.
Figure 31: Le logiciel détermine les valeurs angulaires de l'articulation. Ici pour imager, la pente condylienne obtenue par le mouvement de propulsion. Le logiciel suscité ne sortira que la courbe de déplacement.
Figure 32: Boitiers condylien réglables pouvant recevoir les informations de déplacement du condyle récolté avec notre système. A gauche la version numérique, à droite la version mécanique.

Un système de FAO (fabrication assistée par ordinateur) de dispositifs inter occlusaux au sein d'un modèle numérique du massif facial, associe :
- une tomographie volumique à faisceau ouvert, conique - Cône Beam Computerized Tomography ou CBCT- des bases osseuses (mâchoires), qui donne une image de l'os de la mâchoire, et donc de l'articulation de la mandibule
- un scanner optique pour obtenir une vue 3D (c'est à dire en trois dimensions) des arcades dentaires (ensemble de dents de la mâchoire)
- un dispositif de captation de mouvement, ou « motion capture », qui enregistre les déplacements de la mandibule, et remplace avantageusement l'articulateur mécanique, qui fonctionne à partir du moulage en plâtre des deux mâchoires, et se trouve moins précis. Ce dispositif de captation de mouvement travaille à partir de capteurs passifs réfléchissants, ou actifs avec cellules photo sensibles, ou sur la base d'un accéléromètre captant le mouvement de la surface sur laquelle il est posé, ou enfin par centrale inertielle,
- une caméra optique 3D apte à relever dans l'espace les points caractéristiques du massif facial, pour placer les arcades dentaires par rapport à ce massif facial.

On obtient par tomographie volumique une vue 3D des bases osseuses, afin d'obtenir une étude céphalométrique, d'où on déduit une position idéale des dents au niveau du massif facial (partie squelettique de la face : on a une information 3D de la forme et de la longueur des racines et de leur positionnement initial).

Un scanner optique permet d'obtenir une vue 3D des arcades dentaires, et grâce au logiciel de modélisation qui accompagne le système, on peut fusionner la partie « couronnes » avec la partie « racines ».

On peut ainsi obtenir un découpage de chaque dent et les visualiser une par une, puis un set up final avec la position de chacune des dents dans son environnement, puis des modèles intermédiaires, tandis que le logiciel permet de quantifier le déplacement de chaque dent prévu par le traitement orthodontique, dans les trois directions.

L'intérêt de cette modélisation tridimensionnelle est de pouvoir guider l'orthodontiste dans ses choix thérapeutiques : on peut calculer exactement les forces nécessaires à appliquer sur les couronnes pour obtenir un déplacement dans le temps.

En outre dans le cas de la pose de mini vis, afin de renforcer l'ancrage, la modélisation permet de placer ces ancrages corticaux sans risque de télescopage avec les racines et leur déplacement au cours du traitement

L'obtention des données tomodensitométriques des patients peut se faire grâce à tous les scanners médicaux. Le praticien qui ne détient pas de cone beam dans son cabinet peut adresser son patient vers un radiologue qui lui fournira en retour les coupes scanner au format DICOM (Digital imaging and communications in medicine : norme standard pour la gestion informatique des données issues de l'imagerie médicale). Mais il est dans l'intérêt du praticien de posséder son scanner Cone beam. Déjà pour le faible taux de rayons que cela engendre sur le patient mais également et cela pour des raisons pratiques, pour conserver le patient dans les murs du cabinet. Aussi pour un gain de temps et réduire les coûts, il faut limiter le nombre de collaborateurs.

Le procédé selon l'invention est capable de faire fusionner le modèle 3D des arcades dentaires du patient aux données 3D du massif facial du patient issu du scanner. Pour cela, deux possibilités s'offrent à nous :
- Il est possible de s'adresser à son prothésiste dentaire si celui-ci possède un scanner optique 3D. Le praticien doit au préalable réaliser les empreintes des arcades grâce à un matériau à déformation plastique. Il récupère ensuite le fichier numérique des arcades dentaires après l'envoi chez son prothésiste de cette empreinte.
- Mais comme cité auparavant, il est dans l'intérêt du praticien de posséder un outil pour numériser des arcades dentaires. Il y a actuellement sur le marché suffisamment d'offres dans cette technologie pour que le dentiste puisse trouver celles qui lui convient. La solution la plus séduisante est d'avoir une caméra d'empreintes optiques intrabuccale au fauteuil.

Comme indiqué plus haut, nous cherchons à obtenir les données de déplacement dans le temps et dans l'espace de la mandibule. Ces informations peuvent venir de différents systèmes comme :
- des axiographes électroniques modernes, qui transforment le mouvement en données numériques intégrables au logiciel.
- des caméras optiques, qui suivent le déplacement de marqueurs placés sur la surface des dents
- de l'évolution de déplacement entre plusieurs capteurs répartis au niveau des arcades dentaires supérieure et inférieure. Les capteurs utilisés sont des capteurs inertiels ou des accéléromètres.

Les informations de déplacement sont ensuite prises en compte par le modèle 3D.

Le logiciel va traiter l'ensemble des données que l'on va apporter. C'est à la fois un logiciel d'analyse céphalométrique, à la fois un logiciel de modélisation et de rendu de surface. C'est également un logiciel qui matérialise le traitement par la conception de bracket par FAO et aide le praticien à la mise en fonction de son traitement en bouche.

Nous allons décrire pas à pas la manipulation des données d'entrée et la façon dont celle-ci sont analysées et travaillées dans le logiciel :
La céphalométrie est l'ensemble des techniques de mesure de la tête. Elle est nécessaire pour le diagnostic en orthodontie. Le praticien en tire des conclusions vis-à-vis d'un possible défaut de positionnement dentaires, mais également d'un défaut de croissance des os maxillaires.

Les données tomodensitométriques peuvent être lues en 2D (deux dimensions) et l'étude céphalométrique peut se faire dans ce sens. Nous y situons les points d'intérêt nécessaires au calcul des angles entre les différents plans de référence. Mais l'intérêt est de faire appel à la tomodensitométrie volumique et d'appliquer un procédé de céphalométrie volumique. Celle-ci offre en plus la possibilité de mettre en évidence les possibles défauts de croissance du sens transversal. Dans tous les cas le procédé selon l'invention s'appuie nécessairement sur la modélisation 3D du massif facial. Le logiciel utilise les données au format DICOM issues de l'imagerie pour recréer en 3D une représentation virtuelle du crâne. A ce stade il est nécessaire d'individualiser l'os mandibulaire du maxillaire ainsi que les racines dentaires de l'os alvéolaires maxillaires et plus précisément les dents avec leurs racines entre elles. Les points de repères sont situés sur le volume 3D du crâne, puis le logiciel nous fournit des informations sous formes de valeurs concernant le positionnement dentaire ou le positionnement des os maxillaires par rapport à la base du crâne. Ces données se basent par rapport à une normalité définie par les auteurs ayant mis en place ce genre d'analyse céphalométrique. La position idéale des dents est alors connue et utilisée dans la suite des opérations.

Il suit alors une étape de création de set up complètement numérique, avec prise en compte du volume radiculaire.

Cette étape découle de l'analyse précédente. La technique qui sera décrite ci-dessous est propre au procédé selon l'invention et n'est retrouvée dans aucun autre logiciel.

Le fichier des arcades dentaires numérisées issu du scanner optique est importé. Ensuite, il faut combiner, corréler, ce modèle 3D aux formes des dents obtenues grâce à l'examen tomodensitométrique. Cela est nécessaire car les arcades obtenues par le système optique ont une meilleure définition. Chaque couronne des dents sur ces arcades est individualisée et il leur est associé les racines leur correspondant. Chaque unité couronne racine est alors déplacée de façon idéale grâce à un « Trackball » (outils du logiciel permettant de déplacer un objet 3D dans l'espace) en se basant par rapport aux résultats de la céphalométrie. Une autre fonction pour déplacer les dents est envisagée. Des courbes en 3D schématisant le positionnement des bords occlusaux des dents ainsi que la position extrêmes des faces vestibulaires des dents sont dessinées. Le milieu inter incisif est également placé. Des points de référence sont situés sur les dents et ces derniers fusionnent avec les courbes préalablement déterminées. Un ajustage grâce au Trackball est ensuite entrepris afin de placer au mieux les racines au sein des bases osseuses mais également d'éviter les proximités radiculaires. L'anticipation des déplacements dentaires grâce à ce set up, véritable simulation du traitement final, permet de maîtriser les forces appliquées afin d'éviter les fenestrations osseuses.

Le procédé selon l'invention se propose de concevoir des appareils multi bagues et multi attaches. La technique proposée de mise en place peut être une technique vestibulaire ou une technique linguale. L'appareil est composé de différents éléments. Notre procédé offre une aide totale ou partielle dans leur mise en forme.
- Les brackets sont des artifices collés à la surface de la dent. Leur rôle est de fixer le fil à mémoire de forme et ainsi de guider la traction orthodontique. Le design ainsi que le placement sur la dent est pris en charge par le logiciel CAO (pour Conception Assistée par Ordinateur). Chaque bague ou attache est munie d'une gorge dans laquelle un fil métallique ou arc orthodontique est glissé. C'est cet arc qui guide le déplacement des dents. Les bagues d'orthodontie, placées correctement, permettent de déplacer les dents avec une grande précision. Cette CAO est ensuite confiée à des machines à commandes numériques pour en assurer leur fabrication. La technique peut être une mise en moufle du bracket imprimé en cire calcinable, ou un usinage par une micro fraiseuse robotisée ou encore une mise en forme par technique de micro fusion de poudre métallique.
- Les arcs : Ils sont choisis en fonction du périmètre d'arcade mais également de la force que l'on veut appliquer. Le projet thérapeutique sous forme de simulation ou set up dirige plus facilement notre choix vis-à-vis de la dimension de l'arc mais également de sa force afin d'éviter les effets secondaires comme les rhizalyses ou fenestrations osseuses. Une fois l'arc adéquat choisi nous proposons un pliage virtuel qui sera ensuite confié à un robot qui assurera le pliage automatique.
- Les minivis : Pour augmenter l'ancrage, nous faisons quelques fois appel à l'utilisation de minivis implantée au niveau de l'os. Notre logiciel favorise le placement de ces minivis puisque la position finale des racines est connue grâce à la simulation. Pour l'implantation, l'expérience du chirurgien-dentiste peut suffire, sinon nous proposons de concevoir des guides chirurgicaux, sorte de petites gouttières à placer à la surface des dents et pré perforées afin de guider la main du chirurgien qui viendra visser ces minivis au sein de l'os.

Le procédé selon l'invention concerne encore la dentisterie restauratrice et l'occlusodontologie. Nous nous intéressons principalement aux procédés de recueil de la cinématique mandibulaire ainsi qu'à sa reproduction.

Les principaux systèmes de suivi des déplacements mandibulaires existants sur le marché ne sont autres qu'une évolution des mandibulographes mis sur le marché dans les années 50. Ces systèmes fixés à la mandibule du patient permettent d'objectiver les déplacements dans l'espace de cette mandibule. Des tracés de ces déplacements sont obtenus et ensuite analysés afin de diagnostiquer une pathologie articulaire ou encore pour programmer un simulateur de la cinématique mandibulaire. Sur ces simulateurs sont placées les arcades dentaires du patient permettant au praticien et au prothésiste de faire un diagnostic puis de réaliser des artifices prothétiques ou des orthèses occlusales. Ces travaux sont ensuite placés dans la bouche du patient afin de rétablir une fonction manducatrice déficiente à cause de la perte des dents ou de soulager des muscles ou une articulation douloureuse grâce à l'orthèse occlusale.

Notre projet est innovant sur plusieurs points. Tout d'abord cette démarche commençant par le recueil de la cinématique mandibulaire jusqu'à la conception des artifices prothétiques est entièrement dématérialisée grâce à l'aide des technologies numériques. Cela se fait en associant les données fournies par différentes technologies comme les dispositifs de captation de mouvement, l'imagerie médicale à Rayons X, ainsi que l'imagerie 3D obtenue à partir de scanners optiques. Ces données sont ensuite traitées et assimilées dans le logiciel, puis les prothèses et orthèses sont fabriquées par des machines-outils à commande numérique.

Nous allons décrire la prise en main de l'interface afin de comprendre son fonctionnement.

Nous pouvons utiliser le logiciel du procédé selon l'invention de différentes façon en fonction du résultat que nous voulons obtenir.

Nous pouvons l'utiliser comme occluseur fonctionnel, c'est-à-dire une association d'arcades dentaires 3D avec un dispositif de captation de mouvement.

Nous faisons appel à cette fonction :
- Lorsque nous voulons remplacer des dents manquantes ou les restaurer par des couronnes ou les restaurer par des inlay/onlay. La position des marqueurs enregistrant le déplacement doit être retrouvée de façon commune entre les modèles 3D des arcades et leur situation réelle en bouche. Cela se fait à l'aide des empreintes physiques ou optiques. La connaissance de l'évolution de la position des capteurs ou marqueurs dans l'espace est appliquée à leurs représentations graphiques virtuelles alors solidaire des arcades dentaires. Par ce fait, nous obtenons la mise en mouvement des arcades virtuelles. Cette fonction n'est applicable que dans des situations cliniques particulières. La présence de guidages dentaires fonctionnels est nécessaire. Dans ce cas, le déplacement de la mandibule est sous l'influence des dents restantes. Nous n'avons pas besoin de prendre en considération la morphologie articulaire.

- La CAO des pièces prothétiques se fait de façon à ce que la morphologie occlusale s'intègre parfaitement dans l'espace fonctionnel offert par la cinématique mandibulaire. La morphologie occlusale de nos restaurations est optimisée et s'intègre sans heurt ni inconfort.
- Lorsque nous voulons concevoir une orthèse occlusale de libération occlusale ou d'antéposition mandibulaire. Nous plaçons la mandibule du patient dans la position de référence et les données de déplacement sont transmises aux modèles 3D. La conception assistée par ordinateur des orthèses s'interposant entre les arcades est alors possible. La fabrication assistée par ordinateur est entreprise grâce à des machines-outils à commandes numériques. Le montage des modèles physiques sur un simulateur mécanique est évité.

Nous pouvons ensuite utiliser le logiciel comme Articulateur physiologique :
Dans certaines situations, nous cherchons à comprendre le fonctionnement de l'articulation parce que celle-ci renferme l'historique de la cinématique mandibulaire surtout lorsque nous avons perdu toute information occlusale. La conception de nos futures prothèses doit se baser sur la morphologie articulaire afin d'éviter la création d'interférences et de prématurités susceptibles de léser l'articulation.

Pour ce faire il faut appliquer les données de l'acquisition de mouvement au modèle virtuel de la mandibule du patient. Comme expliqué précédemment la tomodensitométrie volumique est nécessaire pour la modélisation des structures osseuses. Le logiciel comprend un algorithme permettant d'isoler l'os mandibulaire de la base du crâne. L'examen radiographique peut être passé avec le système d'acquisition en bouche. Il faut alors modéliser les marqueurs ou émetteurs du système de mocap (motion capture, pour dispositif de captation de mouvements) en même temps que la mandibule. Les mesures de déplacement sont appliqués ensuite à la représentation virtuelle des marqueurs. La mandibule virtuelle est alors mise en mouvement. Un seul examen radiographique est nécessaire. Il est possible ensuite de faire autant d'acquisitions de mouvement que l'on veut à condition de placer les marqueurs ou capteurs dans la même situation qu'à l'origine. Une légère variation peut être tolérée car un calcul par transformation rigide est mis en place dans le logiciel. L'acquisition du mouvement doit obligatoirement commencer dans la même position de référence que celle utilisée lors de l'examen radiographique.

Les arcades dentaires virtuelles du scanner optique sont ensuite corrélées sur le modèle issu de la tomodensitométrie. La CAO des travaux de restauration, ainsi que les orthèses occlusales, se font en visualisant en parallèle l'évolution de l'interligne intra articulaire, gage de la bonne santé de l'Articulation Temporo Mandibulaire (ATM).

Nous pouvons enfin utiliser le logiciel comme enregistreur ATM.

Cette fonction nécessite la même manutention que précédemment mais les modèles des arcades ne sont pas intégrées car seule la cinématique mandibulaire nous intéresse. L'intérêt est principalement diagnostique mais peut être aussi thérapeutique dans les manipulations de recoaptation condylodiscale.

Lorsque l'organe dentaire est dégradé, le rôle du chirurgien-dentiste sera de le restaurer. Lors d'un délabrement important, il devra faire appel à des artifices prothétiques remplaçant tout ou partie d'une ou des dents concernées. Cela ne peut se faire sans prendre en considération l'occlusion dentaire qui est la façon dont s'organisent les contacts interdentaires et inter-arcades. Mais ce ne sont pas les seules situations ou la gestion des contacts dentaires est un impératif thérapeutique. Par exemple, lorsque les dents sont en position ectopiques un traitement orthodontique est entrepris. Les dents sont déplacées grâce à un appareil. Le schéma et la distribution des contacts occlusaux changent et des règles sont à respecter dans ce cas pour ne pas nuire au patient. Dans d'autres cas, lorsque des troubles de l'articulation ou de contraction musculaire sont diagnostiqués et ont un rapport avec l'occlusion le chirurgien-dentiste peut par la conception d'une gouttière occlusale atténuer voir corriger ces dysfonctionnements.

Par extension, l'occlusion assure l'interface entre les deux maxillaires. Leur confrontation est possible grâce à un os mobile : la mandibule (maxillaire inférieur). La qualité de cette occlusion est essentielle et doit assurer 3 fonctions essentielles « Centrage ; Calage ; Guidage » de cette même mandibule afin de préserver les structures environnantes (articulation, muscles...) . Cette motilité est donc due à une articulation, l'articulation temporo-mandibulaire (ATM) et une mise en mouvement par la contraction des muscles masticateurs. A tous moments, le chirurgien-dentiste est soucieux de la préservation de la bonne santé de ces composants mais également de son rétablissement. En effet, lorsque des pathologies sont objectivées (myalgie, arthropathie) le chirurgien-dentiste peut, par le biais d'une réhabilitation de l'occlusion avoir un effet rétroactif sur les pathologies de l'appareil manducateur.

La construction ou reconstruction de l'occlusion est sous l'influence de certains déterminants. Ce sont des données liées à l'individu ayant une influence sur l'anatomie occlusale. Ils sont importants à appréhender dans certaines situations car l'artisan prothésiste, par leurs maîtrises s'en inspirera pour modeler la surface occlusale des dents. Dans ce cas il existe des outils sur le marché appelés articulateurs qui tentent plus ou moins bien de reproduire la physiologie de l'appareil manducateur.

Pour mieux comprendre le procédé selon l'invention nous allons décrire succinctement les déterminants de l'occlusion et mettre son application en opposition aux autres systèmes disponibles sur le marché. L'innovation découlant de ce procédé de fabrication devrait se révéler.

Les déterminants de l'occlusion sont définis ainsi : ce sont les facteurs pouvant être classés de l'appareil manducateur qui influencent l'occlusion. Ces facteurs sont divisés en deux groupes: ceux qui sont fixes et ceux qui peuvent être modifiés par le remodelage ou le repositionnement des dents. Les facteurs fixes les plus cités sont la distance intercondylienne; l'anatomie de l'articulation temporo-mandibulaire, qui influent la cinématique mandibulaire; le positionnement de l'arcade maxillaire, et la relation intermaxillaire. Les facteurs modifiables les plus cités sont la forme des dents, la position des dents, la dimension verticale, les courbes occlusales, la hauteur des cuspides, et la profondeur des fosses.

Ces déterminants sont interdépendants les uns des autres. Les facteurs modifiables étant ceux concernés par les travaux de réhabilitation du chirurgien-dentiste, la démarche sera de s'intéresser aux autres fixes et de les maîtriser.

Les déterminants fixes peuvent être listés ainsi :
1. Positionnement vertical et horizontal des arcades par rapport au déterminant postérieur
2. Ecartement condylien
3. Positionnement antéropostérieur des arcades par rapport au déterminant postérieur articulaire
4. Déterminant postérieur, articulaire :
   a. Pente condylienne
   b. Angle de Bennett
   c. Mouvement initial de Bennett

Les simulateurs disponibles sur le marché, en fonction de leur sophistication contrôlent plus ou moins bien ces déterminants. Cela au prix d'une programmation fastidieuse, issue d'une manutention coûteuse en temps et source d'erreur au cabinet dentaire. De plus l'intégration d'une partie de ces paramètres (2,3,4) a pour rôle de reproduire la cinématique mandibulaire. Cette reproduction n'est qu'une approche des réels mouvements mandibulaires puisque l'anatomie de l'articulation temporo-mandibulaire est réduite à une addition de valeurs angulaires schématisant dans les plans de l'espace les trajets du condyle mandibulaire. Cela est matérialisé mécaniquement au niveau des boîtiers condyliens du simulateur.

Le procédé selon l'invention est innovant sur ces points. L'intégration des déterminants se fait de façon plus simple et intuitive. Surtout concernant la reproduction de la cinématique. Nous proposons un véritable enregistrement de celle-ci. Une fois intégrée dans le logiciel elle sera rejouée à volonté afin d'animer les modèles numériques des arcades dentaires. Nous pourrions très bien nous affranchir de la récolte des déterminants (2,3,4) mais cela rend notre procédé compatible avec les systèmes existant sur le marché. Concernant l'information fournie par la position des arcades dentaires dans l'espace par rapport aux articulations et au massif facial (1), son intérêt est d'étudier (en cas d'analyse occlusale) et de reconstruire les courbes occlusales. Ces courbes, la courbe de SPEE et la courbe de WILSON qualifient l'organisation intra arcade. De façon simplifiée, ces courbes correspondent à la façon dont s'orientent les surfaces occlusales des dents, leurs cuspides et les bords incisifs dans l'espace. Cela porte à notre connaissance les possibilités de répartition des forces et de rencontre interarcades. Il en découle une analyse sur la forme individuelle de chaque dent, son anatomie propre, la profondeur des sillons, la hauteur cuspidienne, sa position propre par rapport aux autres dents voisines et antagonistes.

Les nouvelles technologies numériques nous offrent des possibilités de traitement et d'analyse inégalées à ce jour et nous ont poussé à mettre au point différents protocoles cliniques. Ces protocoles sont applicables dans les différentes situations cliniques citées précédemment que nous rappellerons au cours de leurs descriptions.

Ces protocoles sont décrits avec trois technologies différentes.
➢ La première technologie est une caméra optoélectronique, tomodensitométrique utilisée pour recueillir le positionnement dans l'espace de diodes actives, petits marqueurs émettant un signal.
➢ La seconde technologie est une caméra infrarouge composée d'une source lumineuse émettant un signal continu de petits points infrarouges. Ce signal est ensuite récupéré par une petite caméra intégrée. Une méthode de triangulation est appliquée pour déterminer la position dans l'espace de la surface réfléchissante.
➢ La troisième technologie est électromagnétique. Les marqueurs sont des bobines donnant une information selon 6 degrés de liberté de leur position. Une source est placée à côté et émet un champ électromagnétique.

La présente méthode ne décrit pas la conception des caméras mais leurs utilisations visant à positionner les modèles numériques d'arcades dentaires par rapport à des points de référence caractérisant le massif osseux de la face. Etape nécessaire dans la conception d'artifices prothétiques, orthèses occlusales, ou encore dans la planification de traitement orthodontique visant à élaborer l'appareil orthodontique. Cette démarche est aussi utile lors de toutes étapes d'analyse occlusale.

Le protocole est divisé en différentes étapes légèrement variables en fonction des méthodes :
✔ Placement des repères, points de référence : Des repères cutanés et d'autres extrapolés par le logiciel sont positionnés au niveau des zones de références caractérisant le massif facial du patient. Permet également la gestion du déterminant 2.
✔ Détermination des plans et axe de référence à partir des points de référence : Un plan horizontal, un plan sagittal médian, l'axe de « rotation » bicondylien
✔ Situation des arcades dentaires par rapport à ces plans. Gestion des déterminants 1 et 3
✔ Etude et enregistrement de la cinématique mandibulaire. Déterminants 4

### I. Placement des repères.

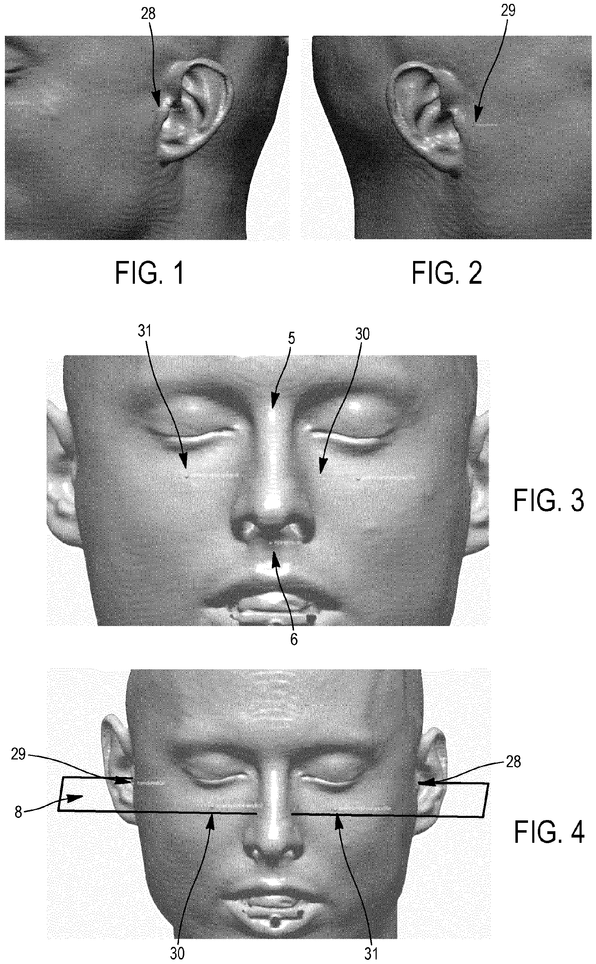

En avant des tragus à droite et à gauche, points correspondants aux condyles. La fig. 1 illustre le condyle gauche 28, la fig. 2 le condyle droit 29.

Sur les points sous orbitaires 30, 31, à la palpation rebord osseux sous l'oeil, à droite 31 et à gauche 30.

Placer un point de couleur, une diode, un repère réfléchissant, ou une bobine électromagnétique sous le nez correspondant à l'épine nasale 6 et/ou sur le point nasion 5.

### II. Détermination des plans de référence à partir des points de référence

- *Créer le plan horizontal, le plan de francfort 8, plan de référence qui représente la base du crâne (il correspondra à la branche supérieur de l'articulateur).*
   Le plan passe par les deux points condyliens 28, 29 et les points sous orbitaires 30, 31
- *Ensuite situer le plan sagittal médian (il correspondra à la symétrie médiane de l'articulateur).*

Calculer le point intercondylien 7 situé au milieu des deux points condyliens. Un algorithme informatique détermine la position spatiale de ce point en se basant sur le positionnement des points condyliens vus par la caméra.

Prendre le point sous nasal ou le point nasion, le plan sagittal 9 passe par l'un des points nasion 5 ou sous nasal 6, et le point intercondylien 7, et est perpendiculaire au plan horizontal de francfort 8.

Ces démarches de positionnement des points de référence sont identiques quelle que soit la technologie employée pour enregistrer la position des repères.

### III. Situer la position des arcades dentaires par rapport à ces plans

### a. Première solution : Utilisation de la caméra vidéo infrarouge.

Une plaque de positionnement 10 est modifiée avec des repères calibrés (ici sous forme de boules 11) pour objectiver la position des dents maxillaires par rapport aux plans : Horizontal de francfort 8 et sagittal 9.

Un repère de positionnement 12 permet de situer le milieu incisif. Il est objectivé en avant sur la plaque par une boule 11 ou une autre forme facilement repérable par la caméra.

Pour rappel, les précédentes images sont issues d'une modélisation mais correspondent à une situation clinique.

Ensuite, l'objectif est d'enregistrer avec un matériau 13 à déformation plastique le placement des dents sur la partie intrabuccale de la plaque. Cette portion est dissociable de la partie extrabuccale pour être scannée. Un butoir interincisif 12 facilite le positionnement et apporte d'autres informations. La distance entre le butoir interincisif 12 et la boule repère 11 est paramétrée et connue du logiciel. La caméra ne pouvant repérer que l'élément externe, le logiciel pourra alors définir le point incisif.

La plaque est composée de deux parties séparables car ses dimensions ne lui permettent pas d'être scannée. La plaque et ses deux parties ont des dimensions connues et intégrées dans le logiciel. Le scanner, lors de l'acquisition 3D de l'empreinte 14 reconnaît également les bords et des repères sur la partie intrabuccale. La situation de la partie extrabuccale est donc déduite. La caméra ayant détecté les repères extrabuccaux l'ensemble est donc placé dans l'espace par rapport aux plans suscités.

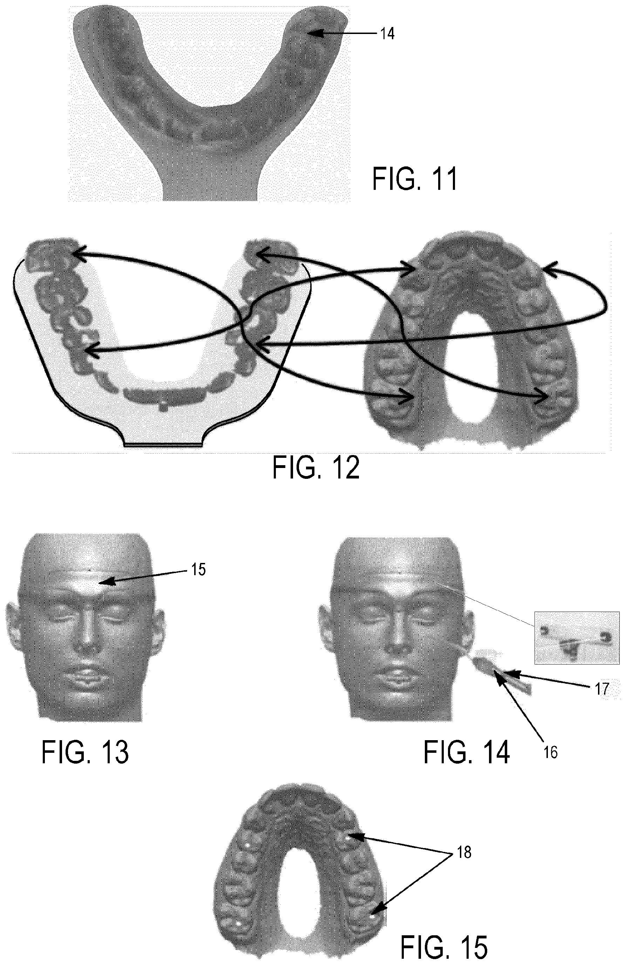

Il est possible ensuite de placer sur le scan de l'empreinte 14 le modèle numérique du modèle maxillaire par corrélation entre les formes (voir fig. 12). L'orientation et la position du maxillaire par rapport à la base du crâne mais surtout par rapport aux deux condyles 28, 29 est alors déterminée. Le modèle numérique de l'arcade mandibulaire vient ensuite en contact du modèle maxillaire, nous ne développerons pas les moyens de mise en relation intermaxillaire.

Avantages : Cette caméra est un véritable scanner grâce à ses capteurs de profondeur capable de transcrire une surface en nuage de points à laquelle est associée une caméra RGB pour ajouter de la texture à l'objet 3D. L'intérêt est d'avoir à disposition l'environnement cutané des lèvres lors de la reconstruction des dents du secteur antérieur. Le projet prothétique virtuel intéressant le secteur antérieur se fera en fonction du bord des lèvres pour situer la ligne du sourire.

### b. Deuxième solution : Utilisation de la caméra active optoélectronique à reconnaissance de diodes actives.

Un autre type de caméra peut être utilisé pour localiser la position de l'arcade maxillaire et par conséquent l'arcade mandibulaire. Elle repère dans l'espace des diodes actives. Le protocole de détermination des plans et des points de référence est légèrement différent. Le système comprend un petit stylet de dimension calibrée et surmonté de trois petites diodes permettant une triangulation et son repérage dans les trois dimensions par la caméra. L'objectif est le même : Pouvoir placer le modèle numérique du modèle maxillaire par rapport aux plans et points de référence caractérisant le massif facial comme le sont les véritables arcades du patient par rapport au massif osseux. Orientation et position sont obtenues.

### Première étape :

### ➢ Placement du bandeau 15 avec le boîtier de diodes sur le front.

Ce boitier est le référentiel de positionnement déterminant toutes les étapes suivantes. Il se plaque sur le front du patient et est maintenu par un bandeau. Un système d'appui nasal assure également la stabilité. Trois diodes minimum réparties sur la surface sont à tout moment visibles par la caméra.

### Deuxième étape :

### ➢ Pointage des points caractéristiques de la face

Cette étape détermine le positionnement des points de références et des plans cités plus haut. Il n'est pas nécessaire d'appliquer sur la peau du patient des diodes actives. L'utilisation d'un stylet 16 est suffisante pour piquer les points sur le visage du patient. L'enregistrement de ces points constitue une image volumique du massif facial. Un bouton 17 sur le stylet permet de cliquer pour valider la position des points de référence à chaque fois que l'extrémité du palpeur est en place sur les points anatomiques correspondants. Le stylet est conçu d'une telle façon que le logiciel détermine la position de la pointe dans l'espace lorsque la caméra traque les diodes disposées sur le manche. A chaque clic, les points de référence sont fixés dans le référentiel du boitier frontal. Pendant l'acquisition l'immobilité est demandée au patient. Mais après la fin du « picking » les mouvements de la tête ne sont plus un problème. Si la tête bouge et donc le boitier, une transformation rigide est appliquée aux différents points pour conserver leur position par rapport au volume facial.

### Troisième étape :

### Pointage des points sur l'arcade maxillaire

Les points 18 à rechercher sont placés de manière aléatoire sur le modèle 3D et cela intéresse aussi bien une arcade partiellement dentée, avec des dents préparées ou des piliers implantaires voire encore une arcade édentée. L'impératif est de placer l'embout du stylet en bouche aux mêmes endroits que sur le modèle virtuel.

Les zones choisies 18 sont des zones qui assurent la stabilité de l'extrémité du marqueur. Ici les fosses des dents sont choisies.

Le stylet 16 placé en bouche, a ses capteurs 19 repérés par la caméra. A chaque clic, la position des points ciblés est vue par la caméra par rapport au boitier frontal. Lorsque les points à cliquer comme sélectionnés sur le modèle 3D ont été situés et validés grâce au stylet, le modèle 3D prend sa place par rapport au référentiel. L'opération préalable ayant permis de connaître la situation des plans et axe de référence par rapport au boitier la position du maxillaire par rapport au plan de référence est déduite. De la même façon, le modèle 3D du maxillaire suit les mouvements du boitier solidaire de la tête.

### Quatrième étape :

### Enregistrement de la cinématique

La position statique du modèle mandibulaire par rapport au maxillaire a été enregistré au préalable en bouche par une caméra optique intrabuccale ou au niveau d'un scanner de table au laboratoire. A cette étape le modèle mandibulaire trouve donc sa place au niveau de la base du crâne et plus particulièrement au niveau des plans et axes de référence la caractérisant. Il faut désormais connaître la façon dont se déplace la mandibule dans l'espace. Le principe est le même que précédemment. Des marqueurs sont placés uniquement sur les dents mandibulaires. La caméra assurera le tracking des diodes placées sur le référentiel frontal et celui des diodes solidaires de l'arcade mandibulaire en mouvement. Le modèle de l'arcade maxillaire et les plans de référence sont associés à l'animation de la mandibule en mouvement. Pour ce faire des boitiers contenant des diodes sont répartis et fixés sur l'arcade mandibulaire. L'empreinte optique servant à restituer en 3D l'arcade mandibulaire est prise à ce moment, les diodes en place (minimum 3). Le modèle 3D comportera deux informations :
➢ La forme de l'arcade et tous les éléments la constituant (par exemple, anatomie occlusale des dents restantes, dents préparées, piliers implantaires, secteurs édentés...)
➢ Le rendu 3d des diodes placées sur l'un de ces éléments.

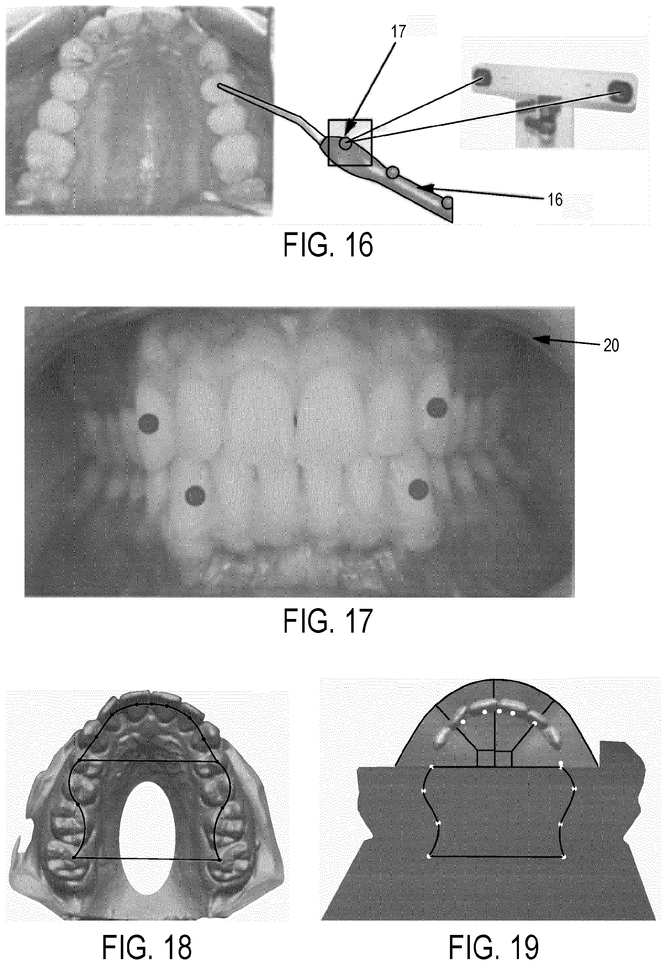

Une corrélation est ensuite assurée entre les mouvements des diodes physiques captées par la caméra et leur modélisation. Ce procédé est nécessaire pour mettre en mouvement l'arcade 3D modélisée. Des écarteurs buccaux 20 sont mis en place pour rendre visibles les diodes par la caméra (voir fig. 17).

Dans certains cas les diodes peuvent être placées en situation buccale pour rendre plus facile la cinématique mandibulaire. Elles sont portées au bout d'une tige dont la taille est connue. Cette tige s'emboite dans une fixation reproductible fixée à une portion de l'arcade. Le scanner de l'arcade est fait à l'identique que précédemment sauf qu'il n'y aura qu'un rendu 3D du système de fixation. Mais la caméra récoltera le déplacement des diodes placées sur la tige à distance du système de fixation. Or, la distance entre les diodes et le système de fixation est connu et invariable. La corrélation entre déplacement des diodes et de l'arcade virtuelle est alors possible.

### Fonctions supplémentaires

D'autres repères sont utiles afin de respecter les règles de l'esthétique. La ligne bipupillaire et le rebord des lèvres sont des éléments sur lesquels le praticien peut s'appuyer pour choisir la longueur des dents du secteur antérieur ou encore choisir la ligne du sourire (des dents) . Un balayage continu avec le stylet sur une surface permet de reproduire en 3D cette surface dans le logiciel
➢ Fonction de dessin des lèvres par balayage avec le stylet. Il est possible de demander au patient de sourire, fixer ce sourire grâce à cette méthode. Puis lors de la modélisation des dents antérieures à reconstruire, le volume en 3D de la lèvre sert de guide.
➢ Ligne bipupillaire. Comme à l'étape de détermination des plans, la pointe du stylet est placée face aux yeux et un clic est fait devant chacun d'entre eux. La ligne est tracée et elle apportera une source d'information supplémentaire afin de respecter un certain parallélisme du plan d'occlusion et de la ligne du sourire par rapport à la ligne bipupillaire.

### c. Troisième solution : Utilisation de marqueurs magnétiques

Ce système peut être utilisé à la fois pour déterminer les plans et points de référence mais aussi pour réaliser la motion capture. Il est composé d'une source et de capteurs.

La source contient des bobines électromagnétiques enfermées dans une coquille en plastique moulé qui émettent des champs magnétiques. La source est la position de référence du système pour les mesures de capteur.

Les capteurs sont des bobines électromagnétiques enfermées dans une enveloppe en plastique moulé qui détectent des champs magnétiques émis par la source. La position et l'orientation des capteurs sont mesurées avec précision lorsqu'ils sont déplacés. Le capteur est un dispositif entièrement passif.

### IV. Les données ainsi obtenues peuvent être exploitées de différentes façons

### a. Pour placer les modèles des arcades dentaires sur notre système avec ses propres algorithmes

La première étape était de déterminer la position statique des arcades. La plaque composée de ses deux parties nous l'a permis tout comme avec le système à diode active et son stylet pointeur ou encore les émetteurs magnétiques Il faut ensuite s'intéresser à la cinématique mandibulaire.

### i. Etude et enregistrement de la cinématique mandibulaire

Pour que notre système soit utilisable, il est nécessaire d'animer le modèle mandibulaire par rapport au modèle maxillaire. La captation du mouvement de la mandibule est essentielle dans ce cas. Deux à trois repères sont placés sur les dents mandibulaires. Or il est nécessaire d'en ajouter sur l'arcade maxillaire car celle-ci, solidaire du crâne peut être soumise à des mouvements parasites dus aux micromouvements de ce dernier même si l'immobilité est demandée au patient. Une transformation rigide est appliquée à tout moment pour transférer ces micromouvements de la tête à ceux de la mandibule, et ainsi compenser ces mouvements involontaires, pour simuler une immobilité parfaite de la tête. Les points cutanés ne sont pas utilisés dans ce cas car la peau est légèrement mobile sous l'action musculaire de mastication.

La caméra n'a pas une définition suffisamment importante pour que nous puissions simplement nous appuyer sur la transmission du mouvement obtenu en bouche au modèle numérique de la mandibule. Dans ce cas, le logiciel, par une autre transformation rigide, applique le mouvement des marqueurs fixés aux dents mandibulaires aux points virtuels matérialisés par les marqueurs situés en regard des condyles. Le tracé obtenu est riche en information puisqu'il caractérise la motilité de l'articulation temporo-mandibulaire(ATM) et peut donc être utile au diagnostic des pathologies articulaires se manifestant souvent par un mouvement anarchique. L'autre point important est que nous pouvons en retirer des données angulaires (angle de Bennett, pente condylienne, déplacement latéral...) donnant une indication sur la forme de l'articulation. Ces données sont aussi transférables vers les simulateurs du commerce (indication développée plus bas) qu'ils soient virtuels ou physiques. La forme de nos restaurations prothétiques en dépendra.

L'enveloppe des mouvements de l'articulation étant connue, elle est réutilisée pour la mise en mouvement du modèle mandibulaire. Un algorithme de détection des collisions entre les deux modèles est intégré au logiciel. Il permet dans un premier lieu d'éviter la pénétration entre les deux maillages mais aussi de marquer dans un code couleur l'intensité et les zones de contacts entre les dents. Pour mobiliser le modèle, il suffit de cliquer sur un point du maillage et de déplacer la souris afin de le mettre en mouvement.

### ii. Une partie du logiciel comprend également toute une partie CAO (conception assistée par ordinateur) propriétaire.

Les premières fonctions concernent la conception de gouttière occlusale.

### 1. REALISATION DE GOUTTIERE (ORTHESE OCCLUSALE) PAR CFAO

### > Modélisation du plan d'occlusion ou plan cuspidien

Sélection des points les plus hauts des dents en postérieur. Créer des courbes réunissant ces points (voir fig. 19). Au niveau antérieur la courbe passe par le milieu des cingulii.

Un plan est créé passant par ces points et courbes.

La surface ici présente sera la limite supérieure de la gouttière, partie en contact avec les dents de l'arcade opposée.

### ➢ Récupération des lignes de profil de la gouttière

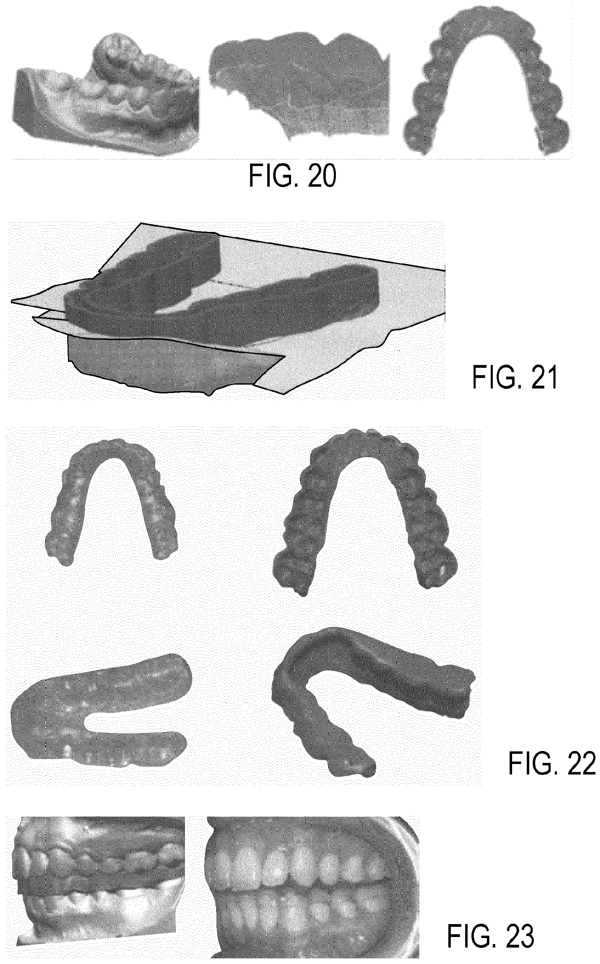

Découper le maillage de l'arcade mandibulaire, et le dupliquer. Les limites de découpe correspondent aux limites de la gouttière (voir fig. 20 à 23).

### 2. ANALYSE OCCLUSALE

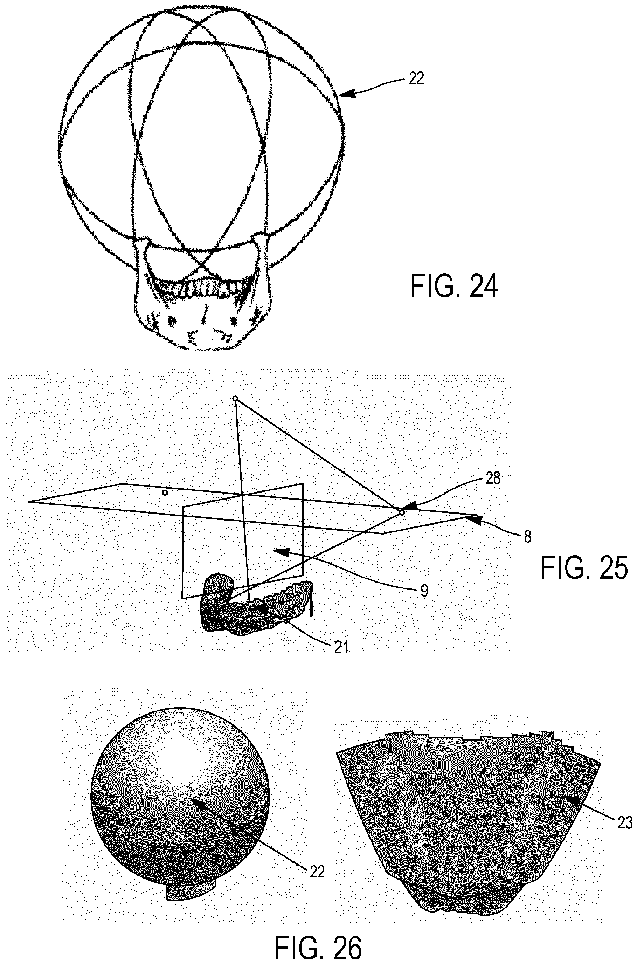

L'orientation des dents au niveau des arcades dentaires se fait d'une manière bien précise. Le plan passant par les cuspides des dents n'est pas plat et est incurvé. En vue sagittale de profil, il est possible de discerner la courbe de Spee, concave vers le bas, passant par la pointe canine et suivant les cuspides vestibulaires des autres dents. En vue frontale de face, la courbe de Wilson, concave vers le haut, si appliquée au maxillaire, réunit le sommet des cuspides vestibulaires et linguales de deux dents homologues (généralement les premières molaires). La Sphère de Monson 22 (voir fig. 24) est décrite ainsi : "La sphère de Monson, dont le centre se situe approximativement au niveau de l'apophyse crista-galli, passe par les pointes cuspidiennes mandibulaires et le versant antérieur du condyle mandibulaire". Elle pourrait à elle seule donner l'orientation de la surface occlusale des dents et donc déterminer la position du plan d'occlusion. Différentes méthodes ont été décrites pour situer le centre de la sphèrede Monson car seul un examen radiographique peut situer l'apophyse crista-galli ; or on cherche souvent à éviter, si possible, les radiographies.

Une première méthode consisterait à déterminer le rayon de courbure de la sphère adapté au patient par l'intermédiaire d'une formule mathématique issue d'une analyse céphalométrique. (JD ORTHLIEB)°

Une autre description situe le centre de la sphère au sommet d'un triangle équilatéral passant par le centre des condyles

Après étude sur un panel d'individus, la méthode décrite par Broadrick est équivalente mais ne tient pas compte du type squelettique du patient. En effet, le rayon de courbure utilisé est toujours le même (10,4 cm) et, par conséquent, ne convient pas systématiquement à tous les patients.

Nous nous inspirerons de la technique décrite par Orthlieb en 1983 et c'est celle-ci qui sera décrite dans notre système. Mais nous ne sommes pas restreints à cette méthode, une évaluation selon le cas clinique est envisagée.

La valeur de la distance entre le dentalé et le condyle est relevée et utilisée pour situer le centre de la sphère sur le plan sagittal médian. Cette valeur est reportée au niveau de la pointe canine 21 et du condyle 28, 29 et le centre de la sphère 22 doit se situer à équidistance de cette valeur par rapport à ces points.

La calotte anatomique 23 ainsi obtenue nous donne une idée de la courbe de Spee en vue sagittale.

Pour l'analyse occlusale les contacts interarcades se marquent en couleur. En position statique et dynamique si nécessaire. Des outils existent pour retoucher le maillage en rapport avec les dents en ajoutant ou supprimant de la matière. Les points de contact évoluent donc. La calotte de la sphère déterminant le plan d'occlusion est utile à ce moment pour évaluer les dents en malposition et les corriger virtuellement. Lorsqu'il manque des dents, l'import se fait à partir d'une banque de données. Ce charting sera utile pour transposer l'ajustement occlusal en bouche. Si la majorité des ajustements sont des ajustements par addition, il est proposé de réaliser une CAO de gouttière ensuite fabriquée en matériau transparent. Elle recevra un matériau composite et sera ensuite appliquée en bouche pour plaquer ce composite sur les dents du patient dans le cas où la dent support ne doit pas recevoir de reconstruction prothétique importante car la perte de substance est minime. Dans les cas où le projet de réhabilitation occlusale indique une perte de substance plus importante, une autre méthode consiste à réaliser une série de couronnes provisoires à la forme de notre modélisation. Une fois les morphologies idéales obtenues, on fait (par exemple par un logiciel adapté) une réduction à minima des futurs piliers dentaires, et conçoit une plaque d'appui muqueuse ou des taquets dentaires de contrôle d'enfoncement. La pièce peut être usinée dans un bloc de PMMA. Ces éléments seront rebasés en bouche pour les adapter aux dents juste après leur préparation. Il est possible de supprimer une ou plusieurs retouches. Instantanément le logiciel peut recalculer les points d'occlusion et ceux-ci peuvent être réaffichés. Il en découle aussi une modification du rapport interarcade avec un possible décalage de l'arcade mandibulaire. On assure son suivi pour mieux déterminer les zones de retouches sur une arcade donnée. Il peut être utile de jouer sur la transparence du maillage pour une meilleure lisibilité des contacts également visibles. A tous moments les mouvements enregistrés peuvent être joués. Durant le mouvement un suivi de la cinématique du l'articulation peut se faire avec l'affichage de la courbe droite et gauche en 2D ou en 3D. Le déplacement est transmis aux arcades.

Pour la thérapeutique occlusale par orthèse des outils sont à disposition afin d'appliquer la méthode décrite précédemment. La gouttière créée est affichée. La spécificité des algorithmes développés est qu'il est possible de sortir du tracé de la courbe. C'est à dire que nous sommes pas obligés de contraindre le déplacement dans certaines situations au déplacement enregistré. Cela est utile lorsqu'une pathologie est diagnostiquée et dont une de ses manifestations est un mouvement incohérent au niveau articulaire. Il peut être utile de disposer également de multiples outils utiles pour concevoir tous types de gouttières (avancée mandibulaire pour traitement des ronchopathie, hyperpropulseurs en orthodontie, gouttière de repositionnement des maxillaires utilisées en per opératoire dans les chirurgie maxillofaciale...). La démarche est pratiquement semblable à celle décrite plus haut. Les éléments communs sont de travailler sur une partie des maillages des modèles placés dans une position donnée.

Pour la thérapeutique orthodontique, l'enjeu est de replacer les dents grâce à un appareil afin que celles-ci soit organisées de façons cohérentes entre elles sur la même arcade et par rapport à l'arcade antagoniste. Le plan d'occlusion est également utile dans ce cas. Les outils disponibles sont des outils permettant de déplacer les dents une par une du genre « trackball ». Des courbes en 3D et inspirées des éléments déjà présents (courbe de Spee, Wilson, plan d'occlusion, et position par rapport à l'axe de rotation articulaire) sont dessinées sur des points de référence (pointes cuspidiennes, bords incisifs, faces vestibulaires). Un algorithme assure ensuite l'alignement entre la courbe faisant défaut et la courbe idéale. Une fois que les points d'occlusion assurant le calage interarcade ainsi que les contacts lors des guidages dentaires sont validés (sous la dépendance du mouvement condylien), d'autres outils assurent la conception de l'appareil d'orthodontie. On obtient des Brackets et fils sur mesure en position linguale ou vestibulaire. Une autre information utile est de connaître le positionnement des racines sous-jacentes des couronnes. Le volume de ces racines est importé, par exemple issu d'un scanner à rayon X. Un algorithme dérivé de l'algorithme des marching cube modélise ces racines qui sont ensuite fusionnées sur les couronnes du set up. Le set up dentaire numérique est quelque fois associé avec une planification chirurgicale mais la chirurgie intervient après le traitement orthodontique.

### 3. ORTHODONTIE ET CHIRURGIE MAXILLOFACIALE

L'orthodontie a pour but de normaliser les rapports dento-alvéolaires pour optimiser la fonction occlusale. Comme nous l'avons vu précédemment l'analyse des fonctions occlusale est pointue et difficile à mener en bouche. Donc, même en orthodontie, le montage des modèles des arcades par rapport aux plans de référence avec possibilité d'étude de la cinématique sont des éléments essentiels. La thérapeutique orthodontique fait souvent appel à l'utilisation d'appareils orthopédiques dont le simulateur virtuel ou physique est le support. En cours de traitement et pour des cas le nécessitant il faut réaliser des gouttières chirurgicales de repositionnement des arcades dentaires lorsque l'os maxillaire ou la mandibule ont été sectionnés.

Avant traitement, l'approche est très semblable à ce qui est décrit pour l'analyse occlusale.

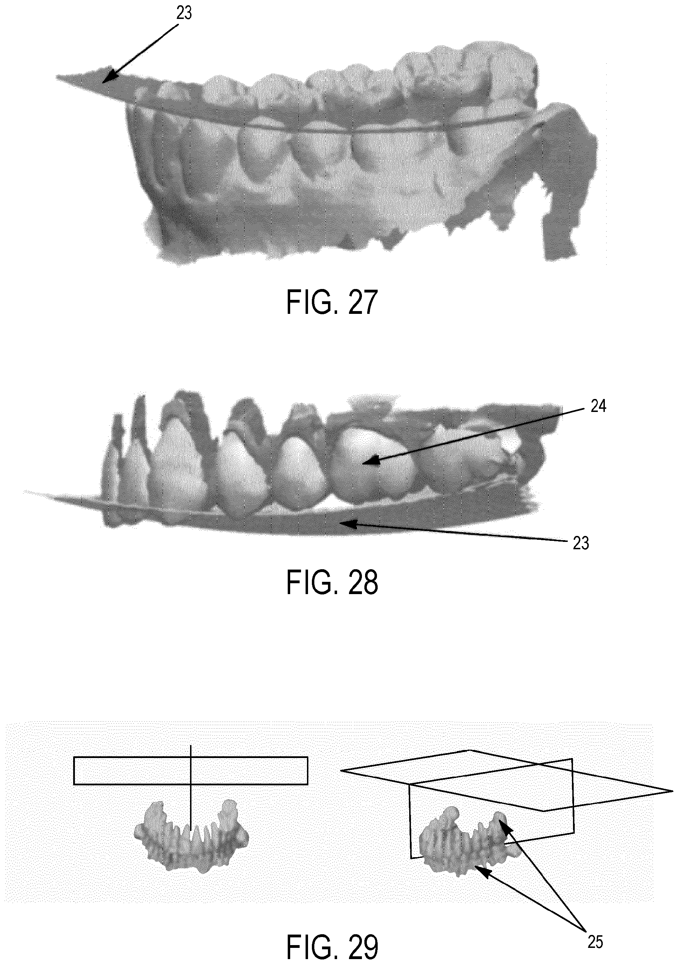

Pour compléter le diagnostic radiographique céphalométrique, il est possible de simuler le déplacement dentaire. La fonction supplémentaire est la fonction « set up ». Cette fonction est rendue très abordable du fait qu'elle se fasse numériquement et qu'elle peut désormais aboutir à la conception de l'appareil orthodontique. Auparavant, cela était réservé à des cas bien précis car la technique nécessitait beaucoup de manutention. Elle peut désormais se démocratiser, et les fonctions telle que le plan d'occlusion 3D rend la technique encore plus fiable. La fig. 28 illustre un set up numérique 24 par rapport à la calotte occlusale 23 dérivée de la sphère de Monson 22.

Si un scanner a été réalisé, il est possible d'importer après reconstruction, les volumes des racines 25 et de les intégrer au set up par corrélation (voir fig. 29). Même si il faudrait plutôt ' importer le set up au sein de la reconstruction des volumes osseux permis par le scanner, cette solution est envisageable. Dans le cas où le scanner est la référence, il n'est plus nécessaire de rechercher les plans de référence par placement des repères cutanés. Des repères osseux sont discernables directement.

Lorsqu'un diagnostic de dysmorphose des bases osseuses est posé, une chirurgie maxillofaciale est entreprise. Il convient de simuler les conséquences de cette chirurgie sur les modèles. Les déplacements maxillaire et ou mandibulaire s'effectuent alors suivant une relation mandibulo-cranienne enregistrée et conservée et selon un plan de référence (le plan axio-orbitaire voisin du plan de francfort) . La quantité et le sens du déplacement des arcades et des structures osseuses les supportant sont déterminés par l'examen clinique et par l'analyse céphalométrique. Les résultats de cette analyse sont importés dans le logiciel pour déplacer les arcades dentaires numériques comme pourrait le faire la chirurgie. Des gouttières modélisables sont réalisées pour assurer le repositionnement du ou des os maxillaires en per opératoire.

Le procédé selon l'invention est très utile pour concevoir des appareils tels que les hyperpropulseurs visant à stimuler la croissance mandibulaire par le phénomène de propulsion mandibulaire. Cette thérapeutique nécessite de connaître la valeur de désinclusion des secteurs molaires, cette valeur étant dépendante de l'articulation et de la pente condylienne. Les données de l'enregistrement de la cinématique mandibulaire étant disponibles, la conception de ce type d'appareil en est facilitée et fiabilisée.

### b. Utilisation des données de positionnement pour assurer le transfert vers les simulateurs virtuels existant dans le commerce.

Nous utilisons les axes de symétrie déterminés par les marqueurs cutanés et le repérage des points condyliens.

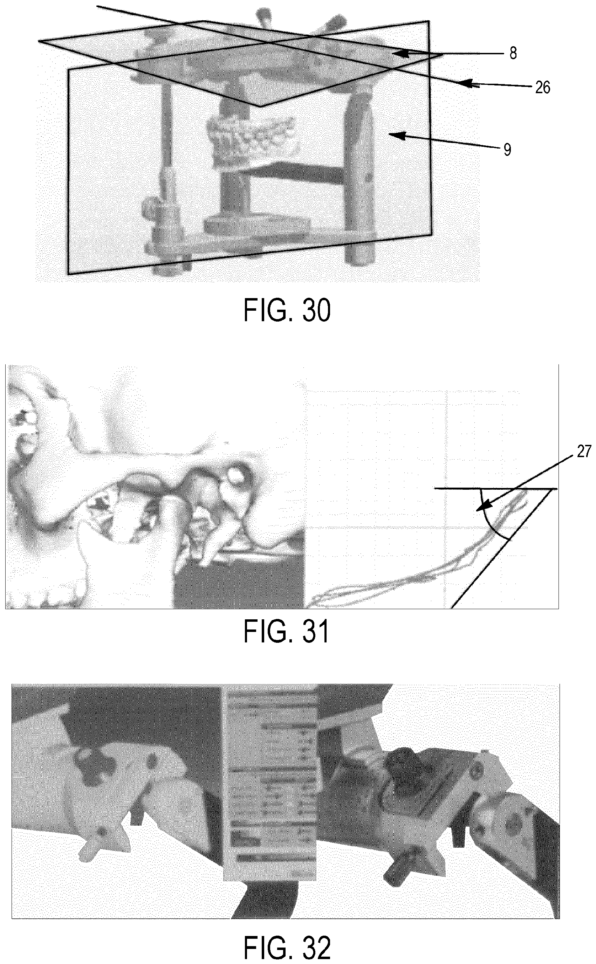

La droite passant par les points condyliens, ou axe bicondylien 26, est centrée sur l'axe charnière de l'articulateur, les modèles sont placés dans le sens antéropostérieur. Le plan de francfort 8 passe au niveau de la branche horizontale de l'articulateur, les modèles sont placés dans le sens vertical. Le plan sagittal 9 passe par l'axe de symétrie de l'articulateur (voir fig. 30) .

Les données angulaires de la forme de l'articulation obtenues à partir de l'étude de la cinématique sont exploitables sur ces systèmes comme peut le faire l'axiographie. L'angle 27 de la pente condylienne est à reporter sur l'articulateur.

L'avantage de la présente invention réside en particulier en ce que la captation de mouvements améliore la définition de l'appareil dentaire, par exemple d'un dispositif inter occlusal (gouttière) mais aussi elle permet d'introduire la possibilité de détecter des pathologies articulaires, et de les traiter précocement grâce à des gouttières construites en FAO, à partir des données numériques recueillies directement sur le patient.

De plus cette modélisation tridimensionnelle permet de guider l'orthodontiste dans ses choix thérapeutiques : on peut calculer exactement les forces nécessaires à appliquer sur les couronnes pour obtenir un déplacement dans le temps.

## Revendications

1. Procédé de conception d'un appareil dentaire destiné à un massif facial (1) comprenant des arcades dentaires, ledit procédé comprenant les étapes suivantes :
- acquisition de points de référence (5, 6, 7) caractérisant le massif facial,
- détermination de plans de référence (8, 9) du massif facial à partir desdits points de référence,
- obtention d'un modèle volumique de chaque arcade dentaire dudit massif facial,
- mise en correspondance des modèles volumiques des arcades dentaires et desdits plans de référence du massif facial,
- enregistrement de la cinématique mandibulaire,
- conception dudit appareil dentaire en prenant en compte l'enregistrement de la cinématique mandibulaire au cours d'une pluralité de mouvements de ladite mandibule, **caractérisé en ce que**
- ladite étape d'enregistrement de la cinématique mandibulaire est effectuée au moyen d'une caméra infrarouge et
- en une dernière étape de production d'un fichier utilisable par des machines à commande numérique aptes à fabriquer un appareil dentaire.

2. Procédé selon la revendication 1, dans lequel l'acquisition des points de référence comprend un pointage de points caractéristiques de la face.

3. Procédé selon l'une des revendications 1 ou 2, dans lequel l'acquisition des points de référence comprend le placement d'un point de couleur, d'une diode, d'un repère réfléchissant ou d'une bobine électromagnétique sur un point de référence de la face à acquérir.

4. Procédé selon l'une des revendications 1 à 3, dans lequel la mise en correspondance du modèle volumique d'une arcade et des plans de référence comprend un pointage, sur ladite arcade, de points sélectionnés sur le modèle volumique de ladite arcade.

5. Procédé selon la revendication 4, dans lequel la mise en correspondance du modèle volumique de l'arcade mandibulaire et des plans de référence est mise en oeuvre à partir d'un enregistrement d'une position statique relative du modèle de l'arcade maxillaire par rapport au modèle de l'arcade mandibulaire.

6. Procédé selon l'une des revendications 1 à 5, dans lequel on met en oeuvre un algorithme de détection des collisions entre les modèles volumiques de l'arcade mandibulaire et de l'arcade maxillaire.

7. Système de conception d'un appareil dentaire d'un massif facial, spécialement conçu pour la mise en oeuvre d'un procédé selon l'une des revendications 1 à 6, et comprenant un ordinateur, et un logiciel apte à traiter les modèles numériques des arcades dentaires et l'enregistrement de la cinématique mandibulaire pour concevoir ledit appareil dentaire, et apte à créer un fichier exploitable par une machine à commande numérique pour fabriquer ledit appareil.

## Patentansprüche

1. Verfahren zur Konzipierung einer Zahnspange, die für einen Gesichtsknochen (1) bestimmt ist, der Zahnbögen umfasst, wobei das Verfahren die folgenden Schritte umfasst:
- Erfassen von Referenzpunkten (5, 6, 7), die den Gesichtsknochen charakterisieren,
- Bestimmen von Referenzebenen (8, 9) des Gesichtsknochens ausgehend von den Referenzpunkten,
- Erhalten eines Volumenmodells jedes Zahnbogens des Gesichtsknochens,
- Zuordnen der Volumenmodelle der Zahnbögen und der Referenzebenen des Gesichtsknochens,
- Aufzeichnen der Mandibularkinematik,
- Konzipieren der Zahnspange unter Berücksichtigung der Aufzeichnung der Mandibularkinematik während einer Vielzahl von Bewegungen der Mandibula,
**dadurch gekennzeichnet, dass**
- der Schritt des Aufzeichnens der Mandibulakinematik mittels einer Infrarotkamera durchgeführt wird und
- in einem letzten Schritt das Erzeugen einer Datei, die von numerisch gesteuerten Maschinen verwendbar ist, die imstande sind, eine Zahnspange herzustellen.

2. Verfahren nach Anspruch 1, wobei das Erfassen der Referenzpunkte eine Ansteuerung charakteristischer Punkte des Gesichts umfasst.

3. Verfahren nach einem der Ansprüche 1 oder 2, wobei die Erfassung der Referenzpunkte das Platzieren eines Farbpunkts, einer Diode, einer reflektierenden Markierung oder einer elektromagnetischen Spule auf einem Referenzpunkt des zu erfassenden Gesichts umfasst.

4. Verfahren nach einem der Ansprüche 1 bis 3, wobei das Zuordnen des Volumenmodells eines Bogens und der Referenzebenen eine Ansteuerung auf dem Bogen von auf dem Volumenmodell des Bogens ausgewählten Punkten umfasst.

5. Verfahren nach Anspruch 4, wobei das Zuordnen des Volumenmodells des Mandibularbogens und der Referenzebenen ausgehend von einer Aufzeichnung einer relativen statischen Position des Modells des Maxillarbogens im Verhältnis zum Modell des Mandibularbogens durchgeführt wird.

6. Verfahren nach einem der Ansprüche 1 bis 5, wobei ein Algorithmus zur Detektion der Kollisionen zwischen den Volumenmodellen des Mandibularbogens und des Maxillarbogens durchgeführt wird.

7. System zur Konzipierung einer Zahnspange eines Gesichtsknochens, das speziell für die Durchführung eines Verfahrens nach einem der Ansprüche 1 bis 6 konzipiert ist, und umfassend einen Rechner und eine Software, die imstande ist, die digitalen Modelle der Zahnbögen zu verarbeiten und die Aufzeichnung der Mandibularkinematik zur Konzipierung der Zahnspange, und imstande, eine von einer numerisch gesteuerten Maschine ausführbare Datei zu erzeugen, um die Spange herzustellen.

## Claims

1. A method for designing a dental appliance intended for a facial mass (1) comprising dental arches, said method comprising the following steps:
- acquiring reference points (5, 6, 7) characterizing the facial mass,
- determining reference planes (8, 9) of the facial mass from said reference points,
- obtaining a volume model of each dental arch of said facial mass,
- matching the volume models of the dental arches and said reference planes of the facial mass,
- recording the mandibular kinematics,
- designing said dental appliance by taking into account the recording of the mandibular kinematics during a plurality of movements of said mandible,
**characterized**:
- **in that** said step of recording the mandibular kinematics is performed by means of an infrared camera, and
- in a last step of producing a file usable by numerically-controlled machines able to manufacture a dental appliance.

2. The method according to claim 1, wherein the acquisition of the reference points comprises pointing characteristic points of the face.

3. The method according to any of claims 1 or 2, wherein the acquisition of the reference points comprises placing a color point, a diode, a reflecting mark or an electromagnetic coil on a reference point of the face to be acquired.

4. The method according to any of claims 1 to 3, wherein the matching of the volume model of an arch and of the reference planes comprises pointing, on said arch, points selected on the volume model of said arch.

5. The method according to claim 4, wherein the matching of the volume model of the mandibular arch and of the reference planes is implemented from a recording of a relative static position of the model of the maxillary arch relative to the model of the mandibular arch.

6. The method according to any of claims 1 to 5, wherein an algorithm is implemented for detecting collisions between the volume models of the mandibular arch and of the maxillary arch.

7. A system for designing a dental appliance of a facial mass, specially designed for the implementation of a method according to any of claims 1 to 6, and comprising a computer, and a software able to process the digital models of the dental arches and the recording of the mandibular kinematics to design said dental appliance, and able to create a file exploitable by a numerically-controlled machine to manufacture said appliance.
